(19) Europäisches Patentamt
European Patent Office
Office européen des brevets

(11) **EP 4 431 058 A1**

(12) **EUROPEAN PATENT APPLICATION**
published in accordance with Art. 153(4) EPC

(43) Date of publication:
**18.09.2024 Bulletin 2024/38**

(21) Application number: **23893748.6**

(22) Date of filing: **17.11.2023**

(51) International Patent Classification (IPC):
***A61F 2/16*** (2006.01)

(52) Cooperative Patent Classification (CPC):
**A61F 2/16**

(86) International application number:
**PCT/CN2023/132377**

(87) International publication number:
**WO 2024/109658 (30.05.2024 Gazette 2024/22)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **22.11.2022 CN 202211470181**

(71) Applicant: **Wuxi Vision Pro Ltd.
Wuxi, Jiangsu 214125 (CN)**

(72) Inventors:
• **LI, Shasha
Wuxi, Jiangsu 214122 (CN)**
• **LIAO, Xiugao
Wuxi, Jiangsu 214122 (CN)**
• **FENG, Zhenyu
Wuxi, Jiangsu 214122 (CN)**

(74) Representative: **Petraz, Gilberto Luigi et al
GLP S.r.l.
Viale Europa Unita, 171
33100 Udine (IT)**

(54) **FULL-VISUAL-RANGE INTRAOCULAR LENS**

(57)    The present invention discloses a full-visual range intraocular lens including an optical body, a first supporting loop and a second supporting loop, wherein the optical body, the first supporting loop and the second supporting loop are of integral structure and are integrally formed from the same material, and the optical body is positioned between the first supporting loop and the second supporting loop; said optical body consists of two optical surfaces, which being spherical or aspherical, and one of the optical surfaces has a diffraction structure for modulating incident optical-field distribution. The present invention adjusts the incident optical-field distribution by means of a diffraction structure, reducing sharp points on the whole optical surface, effectively reducing glare, reducing chromatic aberration, and enabling to see more clearly on the visual range between focal points, so as to bring better visual experience to patients.

FIG. 1

## Description

### Technical Field

The present invention relates to the technical field of optical elements, and in particular to a full-visual range intraocular lens.

### Background Art

Intraocular lens (IOL) refers to an optical element made of synthetic materials, which has become a common medical device to replace the natural lens with turbid lesion, so as to help patients suffering from cataract obtain postoperative vision. Multifocal intraocular lens is a kind of intraocular lens, because multifocal intraocular lens (IOL) can provide two or more focal points (e.g., trifocal IOL provides far-vision, mid-vision and near-vision), and it is widely used for implantation in cataract surgery to replace natural lens of lesion in the eyes of patients. The multifocal IOL can be divided into two categories of diffractive and refractive types, where the surface of the diffractive multifocal IOL includes a plurality of small step gratings with concentric rings to diffract light in several directions, forming a plurality of focal points; the surface of the refractive multifocal IOL may include a plurality of concentric rings of spherical or aspherical surfaces of different curvatures, or a plurality of sectors of spherical or aspherical surfaces of different curvatures, or a combination of both. However, a traditional multifocal IOL still has a problem of discontinuous visual range. There is obvious visual decline or even breakpoint among several focal points. The visual decline or breakpoint can lead to the discontinuity of visual range and the decrease of patient's ability to recognize moving objects.

In summary, how to design a full-visual range intraocular lens to provide a segment of continuous vision for a patient and improve the dynamic vision of the patient is a technical problem to be solved by a person skilled in the art.

### Summary of the Invention

The present invention provides a full-visual range intraocular lens for solving the problem of discontinuous visual range of diffractive multifocal intraocular lens, improving light energy utilization rate, reducing sharp points on the whole optical surface, effectively reducing glare, reducing chromatic aberration, making the full-visual range more clear among focal points, and giving the patients a better visual experience.

To achieve the foregoing purpose, the following technical solutions of the present invention are used:

A full-visual range intraocular lens including an optical body, where the optical body consists of two optical surfaces, said optical surfaces being spherical or aspherical, one of the optical surfaces having a diffraction structure for modulating incident optical-field distribution;

the method for determining an upper optical surface of said optical body includes:

establishing an arbitrary spatial rectangular coordinate system with a vertex of the optical surface as an origin O and an optical axis as an axis Z, where a coordinate axis X of the coordinate system and a coordinate axis Y of the coordinate system are tangent to said optical surface, and a surface shape of said optical surface satisfies the following equation on a Y-Z plane:

$$Z(y) = \frac{cy^2}{1 + \sqrt{1 - (1+K)c^2y^2}} + \sum_{i=m}^{n} A_{2i} y^{2i} \tag{1}$$

where $Z(y)$ is a curve expression of the optical surface on a two-dimensional coordinate system plane Y-Z, $c$ is a reciprocal of a curvature radius of a basic spherical surface of said optical surface, $y$ is a vertical distance from any point on said curve to the coordinate axis Z, $A_{2i}$ is a coefficient of higher-order terms of the optical surface, $m$ and $n$ are both integers not less than 1 and $n>m$, and $K$ is a coefficient of the optical surface; when $K$ and $A_{2i}$ are 0, $Z(y)$ is a spherical equation;

a method of diffraction structure modulating incident optical-field distribution is as follows:

the diffraction structure includes a diffraction ring band structure and a discrete diffraction phase point; firstly, determining a fixed focal point using the diffraction ring band structure, i.e., for a single-focus diffraction element having a diffraction ring band structure, and characterizing the phase thereof by a diffraction phase function $\Phi$:

$$\Phi(x, y) = \rho_1 x^2 + \rho_2 x^4 \tag{2}$$

where *x* and *y* represent longitudinal and transverse coordinates, respectively, in millimeters (mm); $\rho_1$ and $\rho_2$ are various coefficients of the diffraction phase; the phase function $T(\Phi)$ of the diffraction ring band structure is obtained after compressing the diffraction phase function with a period of $2\pi$:

$$T(\Phi) = \Phi - int\left(\frac{\Phi}{2\pi}\right) * 2\pi \quad (3)$$

where *int()* represents a rounding function;

secondly, introducing a discrete diffraction phase point on a sub-wavelength order, where according to Fermat principle, a path of light propagation is the path where an optical length takes the extreme value, which is the maximum value, the minimum value or an inflection point of a function, and is characterized by the formula:

$$OPL = \int_a^b n\left(\vec{s}\right)ds = \frac{\lambda}{2\pi n}\int_a^b d\Phi\left(\vec{s}\right)ds \quad (4)$$

where *OPL* is the optical length; $\vec{s}$ is displacement of light and has a coordinate of $\vec{s}$ (*x, y*), and

$$\left|\vec{s}\right| = \sqrt{(x^2 + y^2)}$$

; $n(\vec{s})$ is a refractive index of the medium at each displacement through which light passes; A is a wavelength of light; $\Phi(\vec{s})$ is a diffraction phase at each displacement through which light passes; when adding a plurality of discrete diffraction phase points $\Phi(\vec{s'})$ of sub-wavelength orders with different positions and sizes in the single-focus diffraction element, formula (4) is converted into the following form:

$$OPL = \frac{\lambda}{2\pi n}\int_a^b \Phi\left(\vec{s}\right)ds + \Phi\left(\vec{s'}\right) \quad (5)$$

in the above formula, *s'* is a coordinate of the discrete diffraction phase point in mm; by adjusting the number, position and size of the discrete diffraction phase point $\Phi(\vec{s'})$ to change the optical length *OPL,* a number of discrete diffraction phase points of different sub-wavelength orders are introduced in the same diffraction ring band structure region, so that the single-focus diffraction element extends from focusing to only one focal point to a range of focal depth with a clear full-visual range, and the method of diffraction structure modulating incident optical-field distribution is applied to the optical body, so that the optical body has the effect of full-visual range.

In the foregoing solution, by introducing the discrete diffraction phase point of sub-wavelength order, the incident optical-field distribution is modulated, the optical length is changed, and then the focal point position within a certain range is changed, and the focal depth range is extended; if a plurality of discrete diffraction phase points with different sub-wavelength orders are introduced in the same diffraction structure region, the single-focus diffraction element can be extended from focusing to only one focal point to a range of focal depth which can be imaged continuously and clearly, and two or even more focal points can be continuously combined to provide a continuous vision for the patient, so as to realize the leap from point vision to continuous vision, improve the dynamic vision of the patient and improve the light energy utilization rate.

Further, said optical body is a lenticular/meniscus lens having a clear optic diameter of 5.5 to 6.5 mm and a central thickness of 0.4 to 1.25 mm.

Further, there are two or more additional focal points of said optical body, the range of the additional dioptric power is +1.5D to +5D, and the full-visual range is +1.5D to +5D.

Said optical body may be a trifocal intraocular lens.

Further, the intraocular lens further includes a first supporting loop and a second supporting loop with said optical body positioned between the first supporting loop and the second supporting loop. Further, said optical body, the first supporting loop and the second supporting loop are of integral structure and are integrally formed from the same material.

Further, the thickness of each of said first supporting loop and said second supporting loop is 0.15 to 0.35 mm.

Further, the surfaces of said first supporting loop and said second supporting loop are both provided with oblique sawtooth grooves or protruding abrazine.

Further, a width of said oblique sawtooth groove or protruding abrazine is 0.2 to 1.0 mm.

Further, a height of said oblique sawtooth groove or protruding abrazine is greater than 40 $\mu$m. Further, an included angle $\alpha$ between an oblique edge of said oblique sawtooth and a plane to which the first supporting loop and the second supporting loop belong is between -20° and +20°.

A full-visual range intraocular lens is prepared through the following steps of design:

(1) Optical design: dividing an intraocular lens into m+n regions in a concentric ring manner, where m is the number of fixed-focus regions (regions where a diffraction ring band structure is located), m+1 is the number of focal points, and n is the number of continuous regions (regions where discrete diffraction phase points are located) (for example, designing a trifocal intraocular lens, and continuously connecting two of the focal points, so that the intraocular lens will be divided into 3-1+1= 3 regions); where the continuous region has a full-visual range effect, and the area of the fixed-focus region accounts for 50%-70% of the whole area of the diffraction structure, and the area of the continuous region accounts for 30%-50% of the whole area of the diffraction structure.

Design of fixed-focus region: according to the design of traditional bifocal intraocular lens, firstly determining the number of additional focal points and corresponding dioptric power, constructing an initial model in Zemax, and then optimizing to obtain the optimal expected effect, obtaining the diffraction basic parameters, and determining a polynomial expression of the diffraction phase function:

$$\Phi_1(x, y) = \rho_{11} x^2 + \rho_{21} x^4$$

$$\Phi_2(x, y) = \rho_{12} x^2 + \rho_{22} x^4$$

$$\cdots$$

$$\Phi_m(x, y) = \rho_{1m} x^2 + \rho_{2m} x^4$$

where $m$ is a positive integer greater than or equal to 2. At the same time, the surface profiles $Z_A$ and $Z_B$ of the two optical surfaces of the intraocular lens are determined; after the diffraction phase function is compressed with a period of $2\pi$, the height $h_{diffraction}$ of the diffraction structure corresponding to each radial position $x$ is calculated; the diffraction ring band structure is superimposed on an optical surface of the optic to obtain an actual optical surface shape $Z_{overall}=Z_{basic} +h_{diffraction}$ of the fixed-focus region, and $Z_{basic}$ is a refractive basal surface.

Design of continuous region: adding a plurality of discrete diffraction phase points $\Phi(\overrightarrow{s_{ni}})$ of sub-wavelength orders with different positions and sizes in each continuous region according to the required dioptric power of the continuous focal point, where $n$ and $i$ are both positive integers greater than or equal to 1, and $i$ is the number of discrete diffraction phase points introduced into one region; $\Phi(\overrightarrow{s_{ni}}) = (x_{ni}, y_{ni})$ is the coordinate of the discrete diffraction phase point in mm.

By adjusting the number, position and size of the discrete diffraction phase points $\Phi(\overrightarrow{s_{ni}})$, introducing these discrete diffraction phase points into formula (5) to change the optical length $OPL$, extending the single-focus diffraction element from focusing to only one focal point to have a range of focal depths that can be imaged continuously and clearly. Similarly, after compressing these discrete diffraction phase points with a period of $2\pi$, the height $h_{continuous}$ of the diffraction structure corresponding to each radial position x can be calculated, and then the actual optical surface shape as $Z'_{overall}=Z_{basic}+h_{continuous}$ of the fixed-focus region can be obtained.

(2) Lathing a base refractive lens: according to the machining parameters of front and rear optical surfaces of optical design, a lathe program is written; an optical body is lathed with a diamond single-point cutting technology; a milling program is written to mill out the profile of optical region and loop feet with abrazine/sawtooth shape; and
(3) Polishing to obtain the intraocular lens with qualified optical surface.

Said full-visual range intraocular lens can be manufactured by firstly machining an intraocular lens disc containing a diffraction structure with a lathe, and then making the lens optical body through mechanical engraving; and the first supporting loop and the second supporting loop are made through mechanical cutting.

Compared with the prior art, the advantageous effects of the technical solution of the present invention are: the present invention extends the focal point by introducing discrete diffraction phase points into the diffraction structure to achieve a continuous visual range, which can not only enable the patient to see clearly on the fixed focal point, but also on the visual range between focal points, so as to achieve a leap from point vision to continuous vision, and solve the problem of discontinuous visual range of the diffractive multifocal intraocular lens.

The optic of the optical body of the present invention has the effect of full-visual range (i.e., continuous visual range), which simulates a continuous zooming function of the human eyes, can clearly image a continuously moving object, improve patients' dynamic vision, and can clearly image the moving object.

In the present invention, discrete diffraction phase points are introduced to adjust the incident optical-field distribution by means of a diffraction ring band structure, reducing sharp points on the whole optical surface, effectively reducing glare and reducing chromatic aberration, so as to bring better visual experience to patients.

In the present invention, the focal points are not completely separated, and the continuous focal depth range increases the light energy utilization rate to greater than 90%, allowing patients to see more clearly in a dark environment.

A one-piece integrally formed full-visual range intraocular lens is simpler in structure than a complicated mechanically adjustable intraocular lens, so that the intraocular lens of the present invention is applicable to a complicated ocular fluid environment, has good stability and is not easy to induce secondary cataract; finally, the design of the abrazine/sawtooth surface of the supporting loops increases the resistance to supporting loop movement and avoids lens rotation within the capsular bag, and further increases postoperative stability.

**Brief Description of Drawings**

Drawings are only for purposes of illustration and cannot to be construed as limiting the present invention. In order to better explain the present embodiment, some components in the drawings are omitted, enlarged or reduced, and do not represent the dimensions of the actual product; it will be appreciated by a person skilled in the art that certain well-known structures and descriptions thereof may be omitted from the figures.

FIG. 1 is a schematic structural diagram showing the front and side structures of the full-visual range intraocular lens of Embodiment 1 according to an embodiment of the present invention;

FIG. 2 is a schematic structural diagram showing the front and side structures of the full-visual range intraocular lens of Embodiment 2 according to an embodiment of the present invention;

FIG. 3 is a schematic structural diagram showing the front and side structures of the full-visual range intraocular lens of Embodiment 3 according to an embodiment of the present invention;

FIG. 4 is a graph of a USAF1951 resolution plate test at different dioptric powers over a continuous range introduced into an eye model for Embodiment 1 according to an embodiment of the present invention;

FIG. 5 is a graph of a USAF1951 resolution plate test at different dioptric powers over a continuous range introduced into an eye model for Embodiment 2 according to an embodiment of the present invention; and

FIG. 6 is a graph of a USAF1951 resolution plate test at different dioptric powers over a continuous range introduced into an eye model for Embodiment 3 according to an embodiment of the present invention.

**Detailed Description of Embodiments**

In order that the objectives, aspects and advantages of the embodiments of the present invention will become more apparent, a more complete description of the embodiments of the present invention will be rendered by reference to the accompanying drawings which illustrate, by way of example, some, but not all embodiments of the present invention. Based on the embodiments of the present application, all other embodiments obtained by a person skilled in the art without involving any inventive effort fall within the scope of protection of the Invention.

Embodiment 1

The present embodiment provides a full-visual range intraocular lens including an optical body 1, where the optical body 1 consists of two optical surfaces, said optical surfaces being spherical or aspherical, one of the optical surfaces having a diffraction structure for modulating incident optical-field distribution;

the method for determining an upper optical surface of the optical body 1 includes:

establishing an arbitrary spatial rectangular coordinate system with a vertex of the optical surface as an origin O and an optical axis as an axis Z, where a coordinate axis X of the coordinate system and a coordinate axis Y of the coordinate system are tangent to the optical surface, and a surface shape of said optical surface satisfies the following equation on a Y-Z plane:

$$Z(y) = \frac{cy^2}{1 + \sqrt{1 - (1 + K)c^2 y^2}} + \sum_{i=m}^{n} A_{2i} y^{2i} \tag{1}$$

where Z(y) is a curve expression of the optical surface on a two-dimensional coordinate system plane Y-Z, $c$ is a reciprocal of a curvature radius of a basic spherical surface of said optical surface, $y$ is a vertical distance from any point on said curve to the coordinate axis Z, $A_{2i}$ is a coefficient of higher-order terms of the optical surface, $m$ and $n$ are both integers not less than 1 and $n>m$, and $K$ is a coefficient of the optical surface; when $K$ and $A_{2i}$ are 0, $Z(y)$ is a spherical equation;

a method of diffraction structure modulating incident optical-field distribution is as follows:

the diffraction structure includes a diffraction ring band structure and a discrete diffraction phase point; firstly, determining a fixed focal point using the diffraction ring band structure, i.e., for a single-focus diffraction element having a diffraction ring band structure, and characterizing the phase thereof by a diffraction phase function Φ:

$$\Phi(x, y) = \rho_1 x^2 + \rho_2 x^4 \tag{2}$$

where $x$ and $y$ represent longitudinal and transverse coordinates, respectively, in millimeters (mm); $\rho_1$ and $\rho_2$ are various coefficients of the diffraction phase; the phase function $T(\Phi)$ of the diffraction ring band structure is obtained after compressing the diffraction phase function with a period of $2\pi$:

$$T(\Phi) = \Phi - int\left(\frac{\Phi}{2\pi}\right) * 2\pi \tag{3}$$

where $int()$ represents a rounding function;

secondly, introducing a discrete diffraction phase point on a sub-wavelength order, where according to Fermat principle, a path of light propagation is the path where an optical length takes the extreme value, which is the maximum value, the minimum value or an inflection point of a function, and is characterized by the formula:

$$OPL = \int_a^b n(\vec{s}) ds = \frac{\lambda}{2\pi n} \int_a^b d\Phi(\vec{s}) ds \tag{4}$$

where *OPL* is the optical length; $\vec{s}$ is displacement of light and has a coordinate of $\vec{s}(x, y)$, and

$$\left|\vec{s}\right| = \sqrt{(x^2 + y^2)}$$

; $n(\vec{s})$ is a refractive index of the medium at each displacement through which light passes; $\lambda$ is a wavelength of light; $\Phi(\vec{s})$ is a diffraction phase at each displacement through which light passes; when adding a plurality of discrete diffraction phase points $\Phi(\vec{s'})$ of sub-wavelength orders with different positions and sizes in the single-focus diffraction element, formula (4) is converted into the following form:

$$OPL = \frac{\lambda}{2\pi n} \int_a^b \Phi(\vec{s}) ds + \Phi(\vec{s'}) \tag{5}$$

in the above formula, $s'$ is a coordinate of the discrete diffraction phase point in mm; by adjusting the number, position and size of the discrete diffraction phase point $\Phi(\vec{s'})$ to change the optical length *OPL*, a number of discrete diffraction phase points of different sub-wavelength orders are introduced in the same diffraction ring band structure region, so that the single-focus diffraction element extends from focusing to only one focal point to a range of focal depth with a clear full-visual range, and the method of diffraction structure modulating incident optical-field distribution method is applied to the optical body 1, so that the optical body 1 has the effect of full-visual range.

Embodiment 2

Specifically, on the basis of Embodiment 1, the solution will be described in conjunction with a specific embodiment to further embody the technical effects of the solution. Specifically:

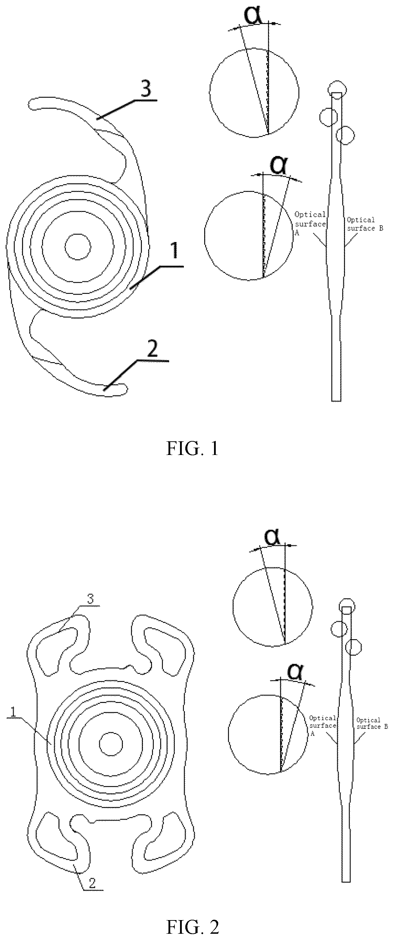

In order to facilitate understanding, with reference to FIG. 1, the present invention provides an embodiment of a full-visual range intraocular lens including an optical body 1, a first supporting loop 2 and a second supporting loop 3, where the optical body 1, the first supporting loop 2 and the second supporting loop 3 are of an integral structure and are integrally formed from the same material, and said optical body 1 is composed of two optical surfaces (an optical surface A and an optical surface B), and said optical surfaces are spherical or aspherical, where the optical surface A has a diffraction structure for modulating the incident optical-field distribution.

The diffraction structure is embossed on an optical surface of the optical body 1 by means of lathing, where the diffraction structure is selected to be superimposed on the optical surface A; the surfaces of the first supporting loop 2 and the second supporting loop 3 are both provided with oblique sawtooth grooves; the height of the oblique sawtooth is greater than 40 μm, the width of the oblique sawtooth groove is 0.2 mm, and the included angle α between the oblique edge of the oblique sawtooth and the plane to which the supporting loop belongs is 20°. The supporting loops include the first supporting loop 2 and the second supporting loop 3.

A lenticular lens has a clear optic of the optical body 1 of 6.0 mm in diameter and a central thickness of 0.67 mm; the thicknesses of said first supporting loop 2 and said second supporting loop 3 are both 0.15 mm; the optical body 1 is made of a hydrophobic polyacrylate having a refractive index of 1.544 and a dispersion coefficient of 45 to 55.

The preparation method for the full-visual range intraocular lens of the present embodiment includes:

(1) Design solution: a trifocal intraocular lens is designed, 20D for far focal point, 2D for middle focal point and 3D for near focal point, and the middle focal point and the near focal point are continued; and

(2) Optical design: the fixed-focus region and the continuous region are divided through each focal length of proposed multifocal points, which are 50 mm (20D), 45.45 mm (22D) and 43.48 mm (23D), as two fixed-focus regions and one continuous region. The total area of the fixed-focus region is 70%, and the total area of the continuous region is 30%. The initial model is built in Zemax, and optimized to obtain the parameters of the optical surface A and the optical surface B of the optical body 1, in which the base radius is 20.5 mm and K=-11.68. The phase functions of the two fixed-focus regions are determined according to the phase coefficients:

$$\Phi_1(x,y) = -101.13x^2 - 0.986x^4$$

$$\Phi_2(x,y) = -151.7x^2 - 1.48x^4$$

Further, according to the required two continuous focal points 22D and 23D, it is decided to add five discrete diffraction phase points, which are uniformly distributed in a continuous region and have a size of intermediate values of $\Phi_1$ and $\Phi_2$.

(3) Lathing the base refractive lens: according to the designed optic parameters, a lathe program is written; a disc intraocular lens is lathed by diamond single-point cutting technology; a milling program is written to mill out the profile of the optic and the supporting loop feet with an abrazine shape.

(4) Polishing to obtain the intraocular lens with qualified optical surface.

(5) Analyzing tests in an eye model.

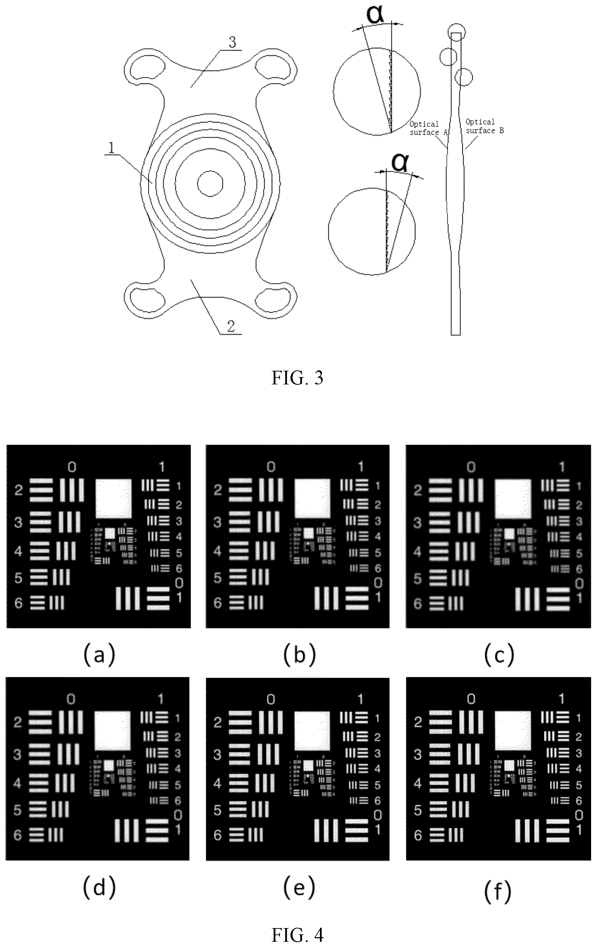

A full-visual range intraocular lens IOL of Embodiment 1 is introduced into the eye model required in ISO11979-2, and a graph of USAF1951 resolution plate test with an interval of 0.2D from 22D to 23D is obtained by means of the optical device test; as shown in FIG. 4, it can be seen that the full-visual range intraocular lens IOL can achieve clear visual effect between 22D and 23D; where a: 22D; b: 22.2D; c: 22.4D; d: 22.6D; e: 22.8D; f: 23D.

In view of the problem that a full-visual range intraocular lens IOL in the prior art has insufficient visual range, obvious visual decline or even breakpoint among several focal points, and the visual decline or breakpoint leading to discontinuous visual range, resulting in insufficient ability of the patient to recognize moving objects, in the present invention, by introducing a discrete diffraction phase point, modulating the incident optical-field distribution, allowing two or even more focal points to be continuous, and measuring MTF values in a continuous range which are all greater than 0.13 in a simulated eye model, it is able to provide a patient with continuous clear vision of 0.6 or more, and achieve a leap from point vision to continuous vision, improving the patient's dynamic vision, clearly imaging the moving objects, and improving the patient's quality of life, at the same time, it has the effects of reducing glare, reducing chromatic aberration and improving light energy utilization rate.

It should be noted that the optic of the optical body 1 of the present invention is a bifocal, trifocal, or multi-intersection optic. The present invention has a better improvement effect on a trifocal IOL for a problem that an obvious discontinuity of visual range in the range from mid-vision to near-vision.

Embodiment 3

Specifically, on the basis of Embodiment 1, the solution will be described in conjunction with a specific embodiment example to further embody the technical effects of the solution. Specifically:

A full-visual range intraocular lens including an optical body 1, a first supporting loop 2 and a second supporting loop 3, where the optical body 1 consists of an optical surface A, an optical surface B and a diffraction structure on the optical surface A. The optical main body 1, the first supporting loop 2 and the second supporting loop 3 are of an integral structure and are formed integrally from the same material; the diffraction structure is embossed on an optical surface of the optical body 1 by means of lathing, where the diffraction structure is selected to be superimposed on the optical surface A.

The surfaces of the first supporting loop 2 and the second supporting loop 3 are provided with several oblique sawtooth grooves, and the width of the oblique sawtooth grooves is 0.2 mm, and an included angle $\alpha$ between the oblique edges of the oblique sawtooth and the plane to which the supporting loops belong is 20°; the height of the oblique sawtooth is greater than 40 $\mu$m.

A lenticular lens has a clear optic of the optical body 1 of 5.5 mm in diameter and a central thickness of 0.6 mm; the thicknesses of the first supporting loop 2 and the second supporting loop 3 are both 0.15 mm; the optical body 1 is made of a hydrophobic polyacrylate having a refractive index of 1.544 and a dispersion coefficient of 45 to 55.

The preparation method for the full-visual range intraocular lens of the present embodiment includes:

(1) Design solution: a trifocal intraocular lens is designed, 15D for far focal point, 2D for middle focal point and 4D for near focal point, and the middle focal point and the near focal point are continued; and

(2) Optical design: the fixed-focus region and the continuous region are divided through each focal length of proposed multifocal points, which are 66.67 mm(15D), 58.82 mm(17D), and 52.63 mm(19D), as two fixed-focus regions and one continuous region. The total area of fixed-focus region is 50%, and the total area of continuous region is 50%. The initial model is built in Zemax, and optimized to obtain the parameters of the optical surface A and the optical surface B of the optical body 1, in which the base radius is 31.57 mm and K=-19.74. The phase functions of the two fixed-focus regions are determined according to the phase coefficients:

$$\Phi_1(x,y) = -101.2x^2 + 0.72x^4$$

$$\Phi_2(x,y) = -202.22x^2 + 1.424x^4$$

Further, according to the required two continuous focal points 17D and 19D, it is decided to add ten discrete diffraction phase points, which are uniformly distributed in a continuous region and have a size of intermediate values of $\Phi_1$ and $\Phi_2$.

(3) Lathing the base refractive lens: according to the designed optic parameters, a lathe program is written; a disc intraocular lens is lathed by diamond single-point cutting technology; a milling program is written to mill out the profile of the optic and the supporting loop feet with an abrazine shape.

(4) Polishing to obtain the intraocular lens with qualified optical surface.

(5) Analyzing tests in an eye model

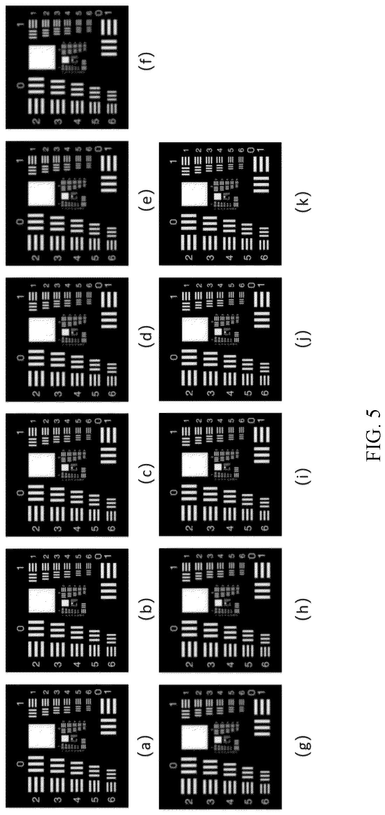

The intraocular lens IOL of the present Embodiment is introduced into the eye model required in ISO11979-2, and a graph of USAF1951 resolution plate test with an interval of 0.2D between the middle focal point (17D) and the near focal point (19D) is obtained by means of an optical device test; as shown in FIG. 5, it can be seen that the full-visual range intraocular lens IOL can achieve clear visual effect between 17D and 19D, where a: 17D; b: 17.2D; c: 17.4D; d: 17.6D; e: 17.8D; f: 18D; g: 18.2D; h: 18.4D; i: 18.6D; j: 18.8D; k: 19D.

Embodiment 4

Specifically, on the basis of Embodiment 1, the solution will be described in conjunction with a specific embodiment example to further embody the technical effects of the solution. Specifically: As shown in FIG. 3, a full-visual range intraocular lens including an optical body 1, a first supporting loop 2 and a second supporting loop 3, where the optical body 1 consists of an optical surface A, an optical surface B and a diffraction structure on the optical surface A.

The optical main body 1, the first supporting loop 2 and the second supporting loop 3 are of an integral structure and are formed integrally from the same material; the diffraction structure is embossed on an optical surface of the optical body 1 by means of lathing, where the diffraction structure is chosen to be superimposed on the optical surface A.

The surfaces of the first supporting loop 2 and the second supporting loop 3 are provided with a plurality of oblique sawtooth grooves, where the height of the oblique sawtooth is greater than 40 μm, the width of the oblique sawtooth groove is 0.2 mm, and an included angle α between the oblique edge of the oblique sawtooth and the plane to which the supporting loop belongs is 20°.

A lenticular lens has a clear optic of the optical body 1 of 5.5 mm in diameter and a central thickness of 0.78 mm; the thicknesses of the first supporting loop 2 and the second supporting loop 3 are both 0.15 mm; the optical body 1 is made of a hydrophobic polyacrylate having a refractive index of 1.544 and a dispersion coefficient of 45 to 55.

The preparation method for the full-visual range intraocular lens of the present embodiment includes:

(1) Design solution: a trifocal intraocular lens is designed, 28D for far focal point, 2.5D for middle focal point and 4D for near focal point, and the middle focal point and the near focal point are continued; and

(2) Optical design: the fixed-focus region and the continuous region are divided through each focal length of proposed multifocal points, which are 35.71 mm(28D), 32.79 mm(30.5D), and 31.25 mm(32D), as two fixed-focus regions and one continuous region. The total area of fixed-focus region is 60%, and the total area of continuous region is 40%. The initial model is built in Zemax, and optimized to obtain the parameters of the optical surface A and the optical surface B of the optical body 1, in which the base radius is 15.75 mm and K=-8.14. The phase functions of the two fixed-focus regions are determined according to the phase coefficients:

$$\Phi_1(x,y) = -128.746x^2 + 0.096x^4$$

$$\Phi_2(x,y) = -206.925x^2 + 0.709x^4$$

Further, according to the required two continuous focal points 30.5D and 33D, it is decided to add eight discrete diffraction phase points, which are uniformly distributed in a continuous region and have a size of intermediate values of $\Phi_1$ and $\Phi_2$.

(3) Lathing the base refractive lens: according to the designed optic parameters, a lathe program is written; a disc intraocular lens is lathed by diamond single-point cutting technology; a milling program is written to mill out the profile of the optic and the supporting loop feet with an abrazine shape.

(4) Polishing to obtain the intraocular lens with qualified optical surface.

(5) Analyzing tests in an eye model

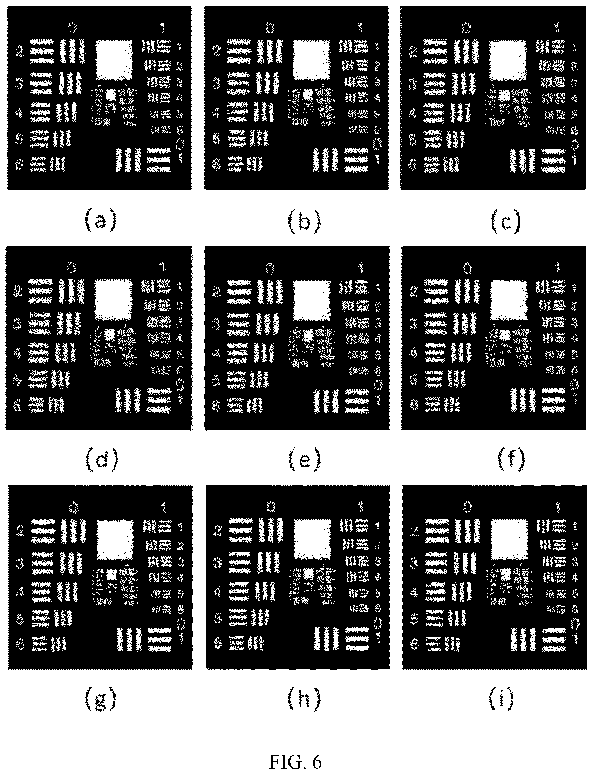

The intraocular lens IOL of the present Embodiment is introduced into the eye model required in ISO11979-2, and a graph of USAF1951 resolution plate test with an interval of 0.2D between the middle focal point (30.5D) and the near focus (32D) is obtained by means of an optical device test; as shown in FIG. 6, it can be seen that clear visual effect between 30.5D and 32D can be achieved, where a: 30.5D; b: 30.6D; c: 30.8D; d: 31D; e: 31.2D; f: 31.4D; g: 31.6D; h: 31.8D; i: 32D.

It can be understood that the above-mentioned embodiments illustrate the implementation effect of the present invention by taking a trifocal intraocular lens as an example, and the method for the present invention for solving the problem of discontinuous visual range of a diffractive multifocal intraocular lens is equally applicable to other diffractive multifocal intraocular lenses.

It should be understood that the above-described embodiments of the present invention are merely illustrative of the present invention for purposes of clarity and are not intended to limit the embodiments of the present invention. It will be apparent to a person skilled in the art that various other modifications and variations can be made in the present invention without departing from the scope or spirit of the present invention. All embodiments no need to be, and cannot be, exhaustive. Thus, it is intended that the present invention cover the modifications and variations of this invention provided they come within the scope of the appended claims and their equivalents.

## Claims

1. A full-visual range intraocular lens, comprising an optical body (1), **characterized in that**, the optical body (1) consists of two optical surfaces, said optical surfaces being spherical or aspherical, one of the optical surfaces having a diffraction structure for modulating incident optical-field distribution;

a method for determining an upper optical surface of said optical body (1) comprises:
establishing an arbitrary spatial rectangular coordinate system with a vertex of the optical surface as an origin O and an optical axis as an axis Z, wherein a coordinate axis X of the coordinate system and a coordinate axis Y of the coordinate system are tangent to said optical surface, and a surface shape of said optical surface satisfies the following equation on a Y-Z plane:

$$Z(y) = \frac{cy^2}{1 + \sqrt{1 - (1+K)c^2y^2}} + \sum_{i=m}^{n} A_{2i}y^{2i} \qquad (1)$$

where $Z(y)$ is a curve expression of the optical surface on a two-dimensional coordinate system plane Y-Z, $c$ is a reciprocal of a curvature radius of a basic spherical surface of said optical surface, $y$ is a vertical distance from any point on the curve to the coordinate axis Z, $A_{2i}$ is a coefficient of higher-order terms of the optical surface, $m$ and $n$ are both integers not less than 1 and $n>m$, and $K$ is a coefficient of the optical surface; when $K$ and $A_{2i}$ are 0, $Z(y)$ is a spherical equation;

a method of diffraction structure modulating incident optical-field distribution is as follows:

the diffraction structure comprises a diffraction ring band structure and a discrete diffraction phase point; firstly, determining a fixed focal point using the diffraction ring band structure, i.e., for a single-focus diffraction element having a diffraction ring band structure, and characterizing the phase thereof by a diffraction phase function $\Phi$:

$$\Phi(x,y) = \rho_1 x^2 + \rho_2 x^4 \qquad (2)$$

where $x$ and $y$ represent longitudinal and transverse coordinates, respectively, in millimeters (mm); $\rho_1$ and $\rho_2$ are various coefficients of the diffraction phase; the phase function $T(\Phi)$ of the diffraction ring band structure is obtained after compressing the diffraction phase function with a period of $2\pi$:

$$T(\Phi) = \Phi - int\left(\frac{\Phi}{2\pi}\right) * 2\pi \qquad (3)$$

where $int()$ represents a rounding function;

secondly, introducing a discrete diffraction phase point on a sub-wavelength order, wherein according to Fermat principle, a path of light propagation is the path where an optical length takes the extreme value, which is the maximum value, the minimum value or an inflection point of a function, and is **characterized by** the formula:

$$OPL = \int_a^b n(\vec{s})ds = \frac{\lambda}{2\pi n}\int_a^b d\Phi(\vec{s})ds \qquad (4)$$

where $OPL$ is the optical length; $\vec{s}$ is displacement of light and has a coordinate of $\vec{s}(x, y)$, and

$$\left|\vec{s}\right| = \sqrt{(x^2 + y^2)}$$

; $n(\vec{s})$ is a refractive index of the medium at each displacement through which light passes; A is a wavelength of light; $\Phi(\vec{s})$ is a diffraction phase at each displacement through which light passes; when adding a plurality of discrete diffraction phase points $\Phi(\vec{s'})$ of sub-wavelength orders with different positions and sizes in the single-focus diffraction element, formula (4) is converted into the following form:

$$OPL = \frac{\lambda}{2\pi n}\int_a^b \Phi(\vec{s})ds + \Phi(\vec{s'}) \qquad (5)$$

in the above formula, $s'$ is a coordinate of the discrete diffraction phase point in mm; by adjusting the number, position and size of the discrete diffraction phase point $\Phi(\vec{s'})$ to change the optical length $OPL$, a number of discrete diffraction phase points of different sub-wavelength orders are introduced in the same diffraction ring band structure region, so that the single-focus diffraction element extends from focusing to only one focal point to a range of focal depth with a clear full-visual range, and the method of diffraction structure modulating incident optical-field distribution is applied to the optical body (1), so that the optical body (1) has the effect of full-visual range.

2. The full-visual range intraocular lens according to claim 1, wherein the optical body (1) is a lenticular/meniscus lens having an effective optical zone diameter of 5.5 to 6.5 mm and a central thickness of 0.4 to 1.25 mm.

3. The full-visual range intraocular lens according to claim 1, wherein there are two or more additional focal points of the optical body (1).

4. The full-visual range intraocular lens according to claim 1, wherein further comprising a first supporting loop (2) and a second supporting loop (3) with the optical body (1) positioned between the first supporting loop (2) and the second supporting loop (3).

5. The full-visual range intraocular lens according to claim 4, wherein the optical body (1), the first supporting loop (2) and the second supporting loop (3) are of an integral structure, formed integrally from the same material.

6. The full-visual range intraocular lens according to claim 4, wherein the first supporting loop (2) and the second supporting loop (3) each have a thickness of 0.15-0.35 mm.

7. The full-visual range intraocular lens according to any one of claims 4-6, wherein the surfaces of the first supporting loop (2) and the second supporting loop (3) are both provided with oblique sawtooth grooves or protruding abrazine.

8. The full-visual range intraocular lens according to claim 7, wherein a width of the oblique sawtooth groove or protruding abrazine is 0.2-1.0 mm.

9. The full-visual range intraocular lens according to claim 8, wherein a height of the oblique sawtooth groove or protruding abrazine is greater than 40 $\mu$m.

10. The full-visual range intraocular lens according to claim 9, wherein an included angle $\alpha$ between an oblique edge of the oblique sawtooth and a plane to which the first supporting loop (2) and the second supporting loop (3) belong is between -20° and +20°.

EP 4 431 058 A1

FIG. 1

FIG. 2

12

FIG. 3

(a)          (b)          (c)

(d)          (e)          (f)

FIG. 4

FIG. 5

(a)          (b)          (c)

(d)          (e)          (f)

(g)          (h)          (i)

FIG. 6

## INTERNATIONAL SEARCH REPORT

| | International application No. |
|---|---|
| | **PCT/CN2023/132377** |

### A. CLASSIFICATION OF SUBJECT MATTER

A61F2/16(2006.01)i

According to International Patent Classification (IPC) or to both national classification and IPC

### B. FIELDS SEARCHED

Minimum documentation searched (classification system followed by classification symbols)

IPC: A61F

Documentation searched other than minimum documentation to the extent that such documents are included in the fields searched

Electronic data base consulted during the international search (name of data base and, where practicable, search terms used)

CNTXT; ENTXTC; ENTXT; VEN; CNKI: 成像, 次波长, 焦点, 焦深, 晶体, 晶状体, 离散, 衍射, 相位, 连续, 透镜, full, vision, lens, phase, focus, depth, length, discrete

### C. DOCUMENTS CONSIDERED TO BE RELEVANT

| Category* | Citation of document, with indication, where appropriate, of the relevant passages | Relevant to claim No. |
|---|---|---|
| PX | CN 115721448 A (WUXI VISION PRO LTD.) 03 March 2023 (2023-03-03) claims 1-10, description, paragraphs [0004]-[0125], and figures 1-6 | 1-10 |
| A | CN 108938144 A (WUXI VISION PRO LTD.) 07 December 2018 (2018-12-07) description, paragraphs [0005]-[0069], and figures 1, 4, and 7 | 1-10 |
| A | CN 107212949 A (WUXI VISION PRO LTD.) 29 September 2017 (2017-09-29) entire document | 1-10 |
| A | CN 108814770 A (NANKAI UNIVERSITY) 16 November 2018 (2018-11-16) entire document | 1-10 |
| A | US 2017219846 A1 (MENICON CO., LTD.) 03 August 2017 (2017-08-03) entire document | 1-10 |
| A | WO 2022177821 A1 (RXSIGHT INC.) 25 August 2022 (2022-08-25) entire document | 1-10 |

☐ Further documents are listed in the continuation of Box C.   ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **20 February 2024** | **07 March 2024** |

| Name and mailing address of the ISA/CN | Authorized officer |
|---|---|
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

INTERNATIONAL SEARCH REPORT
Information on patent family members

International application No.

**PCT/CN2023/132377**

| Patent document cited in search report | | | Publication date (day/month/year) | Patent family member(s) | | | Publication date (day/month/year) |
|---|---|---|---|---|---|---|---|
| CN | 115721448 | A | 03 March 2023 | None | | | |
| CN | 108938144 | A | 07 December 2018 | None | | | |
| CN | 107212949 | A | 29 September 2017 | None | | | |
| CN | 108814770 | A | 16 November 2018 | None | | | |
| US | 2017219846 | A1 | 03 August 2017 | US | 10295841 | B2 | 21 May 2019 |
| WO | 2022177821 | A1 | 25 August 2022 | None | | | |